# EUROPÄISCHE PATENTSCHRIFT

(11) **EP 1 175 500 B1**
(45) Veröffentlichungstag und Bekanntmachung des Hinweises auf die Patenterteilung: **04.08.2004**
(21) Anmeldenummer: 00936699.8
(22) Anmeldetag: 26.04.2000
(51) Int. Cl.: C12P 7/62, C12N 9/20

(54) **VERFAHREN ZUR SELEKTIVEN VERESTERUNG VON POLYOLEN**
METHOD FOR THE SELECTIVE ESTERIFICATION OF POLYOLES
PROCEDE D'ESTERIFICATION SELECTIVE DE POLYOLS

(30) Priorität: 05.05.1999 DE 19920558; 28.05.1999 DE 19924221
(43) Veröffentlichungstag der Anmeldung: 30.01.2002
(73) Patentinhaber: Cognis Deutschland GmbH & Co. KG, 40589 Düsseldorf (DE)
(72) Erfinder: BORNSCHEUER, Uwe, 17489 Greifswald (DE); SCHMID, Rolf, D., 70329 Stuttgart (DE); SYLDATK, Christoph, 70565 Stuttgart (DE); YAN, Youchun, Institut für Technische Biochemie, 70589 Stuttgart (DE); OTTO, Ralf, 74177 Bad Friedrichshall (DE)
(86) Internationale Anmeldenummer: PCT/EP2000/003764
(87) Internationale Veröffentlichungsnummer: WO 2000/068408

(56) Entgegenhaltungen:
- EP-A- 0 337 920
- EP-A- 0 357 009
- DE-A- 19 626 943
- CAO ET AL.: "Lipase-catalyzed solid phase synthesis of sugar fatty acid esters" BIOCATALYSIS AND BIOTRANSFORMATION, Bd. 14, 1997, Seiten 269-283, XP000925239 in der Anmeldung erwähnt

## Beschreibung

Die vorliegende Erfindung betrifft ein Verfahren zur enzymatisch katalysierten Herstellung von Carbonsäureestern mehrwertiger Alkohole.

Auf chemischem Weg hergestellte oberflächenaktive Substanzen sind in der Regel aus Alkyl- oder Arylgruppen aufgebaut, die bei ionischen Tensiden als die Wasserlöslichkeit verstärkende Anteile Carboxylat-, Sulfonat, Phosphat- oder Ammoniumgruppen und bei den nichtionischen Verbindungen Alkohol- oder Polyethergruppen oder Zuckerreste enthalten. Von Vorteil ist bei derartigen Tensiden ihre über viele Jahrzehnte in großtechnischem Maßstab optimierte relativ einfache und preiswerte Herstellung. Ein Nachteil ist die relativ geringe Varianz bei den funktionellen Gruppen im lipophilen Molekülanteil. Als nachteilig wird auch oft empfunden, daß ein Großteil immer noch auf Erdöl als Rohstoffbasis angewiesen ist. Derartige Tenside werden in Lebensmitteln und in Pharmaprodukten daher nur in geringem Umfang eingesetzt. In Wasch - und Reinigungsmitteln sowie in Kosmetika basiert heute mindestens die Hälfte der verwendeten Tenside auf natürlichen Ölen und Fetten. Sogenannte Biotenside zeigen im Gegensatz zu den sogenannten chemischen Tensiden eine große Strukturvielfalt nicht nur im hydrophilen sondem auch im lipophilen Molekülanteil (S. Lang und F. Wagner in: *Biosurfactants and Biotechnology,* Ed.: N. Kosaric, W. L. Caims und N. C. C. Gray, Verlag Marcel Dekker, New York, **1987**, 25, 21-46). Meist handelt es sich um mikrobielle Sekundärmetabolite, die von Produzentenstämmen bevorzugt bei Wachstum auf lipophilen Substraten wie n-Alkanen oder Triglyceriden gebildet werden. Neben guter Umweltverträglichkeit zeigen diese Verbindungen oft auch interessante biologische Effekte wie zum Beispiel Membranaktivität oder Antibiotikawirkung, die sie für die industrielle Anwendung im Pharma-, Kosmetik- und Lebensmittelbereich zunehmend interessant erscheinen lassen. Hier werden bisher fast ausschließlich pflanzliche oder tierische Biotenside verwendet (V. Klekner und N. Kosaric in: *Biosurfactants: Production-Properties-Applications,* Ed.: N. Kosaric, Verlag Marcel Dekker, New York, **1993**, 48, 373-390), die nach aufwendigen Verfahren hergestellt werden. Hier besteht Bedarf nach einfacheren Methoden der Herstellung, welche derartige Substanzen in hoher Ausbeute und Reinheit zur Verfügung stellen.

Die Herstellung von Zuckerestem aliphatischer Carbonsäuren mit Hilfe üblicher Methoden der chemischen Synthese ist bekannt (J.C Colbert, *Sugar Esters - Preparation and Application,* Noyes Data Corporation, New Jersey **1974**). Die chemische Darstellung von Estern aus ungeschützten Zuckern, das heißt Verbindungen mit mehreren frei vorliegenden Alkoholfunktionen, und Carbonsäuren führt in aller Regel zu unspezifischen Gemischen aus ein- und mehrfach acylierten Zuckern, so daß die Einführung und Entfernung von Schutzgruppen notwendig ist, wenn man gezielt ein bestimmtes Produkt synthetiseren will. Durch den Einsatz aktivierter Carbonsäurederivate wie Säurechloriden oder Säureanhydriden entstehen zwangsläufig Beiprodukte und häufig auch unerwünschte Nebenprodukte, welche die Umwelt belasten, die Aufarbeitung erschweren und die Ausbeuten an gewünschtem Produkt vermindern. Auch die Herstellung von Zuckerestern aromatischer Carbonsäuren mit Hilfe derartiger üblicher Methoden der chemischen Synthese ist bekannt (A.F. Artamonov, L. F. Burkovskaya und G. V. Nikonov, *Khim. Prir. Soedin* **1994**, 4, 561-562), wobei die vorstehend genannten Nachteile in gleicher Weise zum Tragen kommen.

Eine ebenfalls in der Literatur beschriebene Methode zur Gewinnung von Estern aus Zuckern oder Glycosiden und aromatischen Carbonsäuren sind Biotransformationen mit Pflanzenzellkulturen (M. Ushiyama, S. Kumagai und T. Furuya, *Phytochemistry* **1989**, 28, 3335-3339). Jedoch werden von diesen Autoren lediglich analytische Ausbeuten beschrieben, da vermutlich durch Abbau- und Weiterreaktionen die Zuckerester schnell wieder in andere Komponenten überführt werden, so daß dieser Zugang wirtschaftlich nicht brauchbar ist.

Die am häufigsten beschriebene Methode zur Gewinnung aromatischer Ester von Zuckern beziehungsweise Glycosiden und aromatischen Carbonsäuren ist die Isolierung aus natürlich vorkommenden Quellen, insbesondere Pflanzen (P.C. Lyons, K.V. Woods und R. L. Nicholson, *Phytochemistry* **1990** 29, 97-101; H. Shimomura, Y. Sashida, M. Oohara und H. Teuma, *Phytochemistry* **1988**, 27, 644-646; Y. Kashiwada, G. L Nonaka, I. Nishioka und T. Yamagashi, *Phytochemistry* **1988**, 27, 1473-1477; M. Nicoletti, C. Galeffi, I. Messana, G.B. Marini-Bettolo, J.A. Garbarino und V. Gambaro, *Phytochemistry* **1988**,27,639-641; Y. Kashiwada, G. I. Nonaka und I. Nishioka, *Chem. Pharm. Bull.* **1984**, 32, 3461-3470). Niedrige Ausbeuten und der Einsatz teilweise hochgiftiger Lösungsmittel erschweren den Zugang zu den Zielverbindungen. Außerdem ist man bei diesem Vorgehen auf die Gewinnung der natürlich vorkommenden Vetreter beschränkt, strukturell auch nur gering abgewandelte Ester lassen sich so nicht erhalten.

In der Natur ist die Bildung derartiger Ester der letzte Schritt eines Biosyntheseweges, der durch verschiedene Enzyme aus der Gruppe der Acyltransferasen katalysiert wird. Diese Enzyme zeigen eine relativ hohe Flexibilität hinsichtlich der Acylgruppe, weisen aber eine sehr strenge Selektivität für das zu veresternde Alkohol-Substrat auf. Von erheblichem Nachteil ist dabei, daß sie stöchiometrische Mengen des entsprechenden Acyl-CoenzymA benötigen, was sie für die *in vitro* Synthese praktisch ungeeignet macht. Dennoch ist die enzymatische Kopplung aliphatischer Fettsäuren an einfache Zucker mit Hilfe derartiger Enzyme beschrieben worden. Das Problem der geringen Löslichkeit und Mischbarkeit von Zucker und Fettsäuren wurde hier durch verschiedene Methoden umgangen: i) Einsatz von polaren Lösungsmitteln wie Pyridin oder Dimethylformamid (J. Chopineau, F.D. McCafferty, M. Therisod und AM. Klibanov, *Biotechnol. Bioeng.* **1988**, 31, 208-214), ii) Einführung von Schutzgruppen wie Isopropylidenacetalen oder Phenylborsäureestern um die Löslichkeit der Zuckerkomponente in organischen Lösungsmitteln zu erhöhen (K. Adelhorst, F. Björkling, S. E. Godtfredsen und O. Kirk, *Synthesis* **1990**, 112-115; C. Scheckermann, A. Schlotterbeck, M. Schmidt, M. Wray und S. Lang, *Enzyme Microb. Technol.* 1995 17, 157-162), iii) Verwendung aktivierter Acyldonoren zur Erhöhung der Reaktionsrate (M. Therisod und A. M. Klibanov, *J. Am. Chem. Soc.* **1986**, 108, 5638-5640), iv) Reaktion in einem weitgehend festen System unter Zusatz geringer Mengen eines organischen Lösungsmittels (L. Cao, A. Fischer, U. T. Bomscheuer und R. D. Schmid, *Biocatal. Biotransform.* **1997**, 14, 269-283).

Die Offenlegungsschrift DE 198 25 943 offenbart ein Verfahren zur selektiven Veresterung von primären OH-Gruppen von Mono-, Di-, und Oligosacchariden mittels Lipasen. Geeignete Substrate wurden beschrieben als einfache Mono- und Dissaccharide wie Glucose. Mannose, Galactose oder Saccharose. Es werden in dieser Druckschrift nur Zuckeralkohole oder solche, die wenigstens eine cyclische Zuckereinheit beinhalten offenbart, iedoch keine weiteren Zuckerderivate als Substrate beschrieben.

Nachteile insbesondere der unter Nr. i) und ii) genannten Verfahren sind die Inaktivierung des Enzyms durch das Lösungsmittel, zusätzlich notwendige Syntheseschritte zur Einführung und Abspaltung von Schutzgruppen, geringe Ausbeuten und der Einsatz von Lösungsmitteln, welche die Verwendung der Reaktionsprodukte in bestimmten Anwendungsbereichen, zum Beispiel dem Pharma- oder Lebensmittelbereich, stark einschränken. Als potentiell nachteilig wurde insbesondere bei dem unter Nr. iv) genannten Verfahren gefunden, daß die Aufarbeitung der Reaktionsprodukte aus einem weitgehend festen Reaktionsgemisch oft nicht verlustfrei möglich ist und zudem bei dieser Verfahrensweise eine kontinuierliche Reaktionsführung erhebliche Schwierigkeiten bereitet.

Überraschenderweise wurde nun gefunden, daß unter Einsatz einer Hydrolase und geringer Mengen eines organischen Lösungsmittels aus Zuckerderivaten und nichtaktivierten Carbonsäurederivaten selektiv entsprechende Ester erhalten werden können.

Gegenstand der Erfindung ist ein Verfahren zur Herstellung selektiv an der primären OH-Gruppe mit Carbonsäuren veresterten Zuckerderivaten, welches dadurch gekennzeichnet ist, daß man das Zuckerderivat ausgewählt aus Aldonsäuren und Ascorbinsäure mit einem Carbonsäureakylester in Gegenwart eines organischen Lösungsmittels unter Katalyse einer Hydrolase, vorzugsweise einer Lipase oder Esterase, umsetzt.

Den zuckerderivaten im Sinne der vorliegenden Erfindung ist zu eigen, daß sie eine primäre Alkoholfunktion und daneben noch mindestens eine weitere, sekundäre oder tertiäre Alkoholfunktion aufweisen. Insbesondere handelt es sich dabei um Ascorbinsäure und Aldonsäuren die von einfachen Zuckerderivaten wie Threose, Erythrose, Arabinose, Lyxose, Ribose, Xylose, Allose, Altrose, Galactose, Glucose, Gulose, Idose, Mannose, Talose und Fructose oder diesen zusammengesetzten Di-, Oligo- und gegebenenfalls Polymeren abgeleitet sind. Die natürlich vorkommenden Isomere der Zucker, in der Mehrzahl die D-Formen, sind bevorzugt. Erfindungswesentlich ist, daß diese Verbindungen neben der für die Veresterungsreaktion notwendigen primären Alkoholgruppe mit mindestens einer freien, das heißt nicht mit einer Schutzgruppe versehenen sekundären oder tertiären Alkoholfunktion eingesetzt werden.

Die mit den genannten zuckern bzw. Zuckerderivaten zu veresternden Carbonsäuren gehorchen vorzugsweise der allgemeinen Formel R-COOH, wobei R ein gegebenenfalls hydroxysubstituierter Alkyl- oder Alkenylrest mit 6 bis 32 C-Atomen oder AR-(CH₂)ₙ ist und AR ein gegebenenfalls alkyl- oder hydroxysubstituierter Phenyl- oder Naphthylrest und n eine Zahl von 0 bis 4 ist. Zu den bevorzugten Vertretern gehören Capronsäure, Önanthsäure, Caprylsäure, Pelargonsäure, Caprinsäure, Laurinsäure, Lauroleinsäure, Myristinsäure, Myristoleinsäure, Palmitinsäure, Palmitoleinsäure, Stearinsäure, Petroselinsäure, Petroselaidinsäure, Ölsäure, Elaidinsäure, Ricinolsäure, Linolsäure, Linolaidinsäure, Linolensäure, Eläostearinsäure, Arachinsäure, Gadoleinsäure, Arachidonsäure, Behensäure, Erucasäure, Brassidinsäure, Clupanodonsäure, Lignocerinsäure, Cerotinsäure, Melissinsäure, Phenylessigsäure, Phenylbuttersäure, Phenylvaleriansäure und meta-Hydroxyphenylessigsäure. Sie werden in Form nichtaktivierter Derivate, insbesondere in Form ihrer Alkyl-, Alkylphenyl- oder Alkenylester eingesetzt, wobei niedere Ester wie Methyl-, Ethyl-, n-Propyl-, iso-Propyl-, n-Butyl-, sec-Butyl-, iso-Butyl-, tert-Butyl- oder Vinylester besonders bevorzugt sind.

Vorzugsweise weicht das im erfindungsgemäßen Verfahren eingesetzte Molverhältnis zwischen dem nichtaktivierten Carbonsäurederivat und dem Zuckerderivat nur möglichst gering von 1 ab und liegt insbesondere im Bereich von 0,8 bis 1,2, da dann die höchsten Ausbeuten an gewünschtem Produkt und die niedrigsten Mengen an Nebenprodukten auftreten.

Normalerweise wird erfindungsgemäß organisches Lösungsmittel in Mengen von etwa 0,1 bis 25 facher, insbesondere 0,5 bis 18 facher Gewichtsmenge an zu veresterndem zuckerderivat eingesetzt, wobei man in einer bevorzugten Ausgestaltung des erfindungsgemäßen Verfahrens die miteinander zur Reaktion kommenden Edukte in einem beide Edukte gut lösenden ersten Lösungsmittel miteinander umsetzt und nach Ende der Reaktion ein zweites Lösungsmittel zusetzt, in dem das entstehende Produkt möglichst wenig löslich ist. Zu den brauchbaren organischen Lösungsmitteln gehören zum Beispiel Dioxan, Acetonitril, Aceton, Ethylmethylketon, γ-Butyrolacton, Tetrahydrofuran, tert.-Butanol, tert.-Amylalkohol und 3-Methyl-3-pentanol sowie deren Gemische, wobei tert.-Butanol ein besonders bevorzugtes erstes Lösungsmittel und Aceton ein besonders bevorzugtes zweites Lösungsmittel ist. In einer bevorzugten Ausführungsform des erfindungsgemäßen Verfahrens wird als nichtaktiviertes Carbonsäurederivat ein Ester, beispielsweise ein Methylester, eingesetzt, der nach Umsetzung mit dem Zuckerderivat einen Alkohol, beispielsweise Methanol, freisetzt, der mittels azeotroper Destillation aus dem Reaktionsgemisch entfernt wird. Bei dieser Verfahrensvariante wird das Lösungsmittel, beispielsweise Aceton, so gewählt, daß es mit dem zu entfernenden Alkohol ein Azeotrop bildet.

Zu den geeigneten Lipasen gehören beispielsweise die aus Candida antarctica, Humicola lanuginosa, Rhizopus spec., Chromobacterium viscosum, Aspergillus niger, Candida rugosa, Penicillium camembertii, Rhizomucor miehei, Burkholderia spec. oder Pseudomonas spec. erhältlichen Enzyme. Vorzugsweise werden sie in fester Form, das heißt in bekannter Weise auf einem Trägermaterial immobilisiert, eingesetzt.

Das erfindungsgemäße Verfahren wird vorzugsweise bei Temperaturen im Bereich von Raumtemperatur bis 80 °C, insbesondere 60 °C, durchgeführt.

Nach Beendigung der Reaktion kann das gewünschte Produkt mit Hilfe üblicher Methoden, zum Beispiel durch Extraktion mit einem geeigneten Lösungsmittel und gegebenenfalls weiterer Reinigung durch beispielsweise Kristallisation oder Chromatographie an Kieselgel, aus dem Reaktionsgemisch isoliert werden.

Das erfindungsgemäße Verfahren erlaubt die chemo- und regioselektive Synthese eines breiten Spektrums bisher nur schwer zugänglicher oder überhaupt noch nicht beschriebener organischer Verbindungen, welche für die Anwendung im Kosmetik-, Lebensmittel-, Pharma- und Umweltsektor von Interesse sind.

Im Hinblick auf den oben zitierten Stand der Technik, insbesondere basierend auf Erfahrungen mit chemischen Reaktionen, mußte man erwarten, daß die Herstellung aus ungeschützten Zuckerderivaten und Fettsäurederivaten wie Fettsäureestern zu unspezifischen Gemischen aus mono- bzw. polyacylierten Zuckerestem führen sollte, verbunden mit den oben genannten Nachteilen. Desweiteren wurden mittels der erfindungsmäßigen Umsetzung Bedingungen entwickelt, welche auch die Umsetzung empfindlicher Substrate wie Vitamin C ohne Zerstörung durch Oxidationen - ein typisches Problem bei chemischen Methoden - erlaubt.

Überdies muß betont werden, daß gemäß der erfindungsmäßigen Umsetzung unter nur geringer Variation der Bedingungen eine sehr breite Palette verschiedenster Produkte in besseren Ausbeuten und höherer Reinheit unter schonenderen Bedingungen hergestellt werden kann, als dies gemäß den aus dem Stand der Technik bekannten Verfahren möglich ist.

Die nach dem erfindungsgemäßen Verfahren erhältlichen Produkte weisen Tensidstruktur auf, das heißt sie bestehen aus einem wasserlöslichen hydrophilen und mindestens einem gut fettlöslichen hydrophoben Molekülanteil. Das Größenverhältnis der Molekülanteile zueinander (Hydrophilic-Lipophilic-Balance oder HLB-Wert) und die darin enthaltenen funktionellen Gruppen bestimmen die Tensideigenschaften der jeweiligen Verbindung. Die erfindungsgemäße Umsetzung erlaubt eine sehr breite Varianz in der Verknüpfung unterschiedlicher Bausteine und damit die einfache Herstellung von Verbindungen unterschiedlicher HLB-Werte. Damit können tensidische Emulgatoren sowohl für Wasser-in-Öl- als auch Öl-in-Wasser-Emulsionen - ein Spektrum, welches für die Anwendung im Kosmetik-, Pharma-, Lebensmittel- und Umweltsektor von hohem Interesse ist - dargestellt werden.

Die Grenzflächenaktivität der nach dem erfindungsgemäßen Verfahren hergestellten Verbindungen ist mit derjenigen chemisch oder fermentativ produzierter aliphatischer Zuckerester mindestens vergleichbar. Deutlich hervorzuheben ist die verbesserte Wasserlöslichkeit der erfindungsgemäß erhaltenen Produkte. Sie sind für den Einsatz als Emulgatoren insbesondere für Öl-in-Wasser-Emulsionen wie auch als tensidischer Bestandteil in Wasch- oder Reinigungsmitteln geeignet. Die Beeinflussung der grenzflächenaktiven Eigenschaften ist in einfacher Weise durch die Wahl entsprechender Acyldonoren möglich. Überdies sind die Verbindungen gut biologisch abbaubar.

Die pharmazeutische Wirksamkeit von nach dem erfindungsgemäßen Verfahren herstellbaren Verbindungen ist vielfältig. Biotenside zeigen nachweislich antibiotische Effekte und Membranaktivität. Darüber hinaus bietet die Umsetzung weitere interessante Möglichkeiten, da sie erlaubt, Wirkstoffe einen eher hydrophoben oder mehr hydrophilen Charakter zu verleihen.

So können aromatische Carbonsäuren über die Glykosylierung einer Therapie mittels Infusionen zugänglich gemacht werden. Andererseits können hydrophile Substanzen wie Vitamin C oder Glykoside wie Salicin mit hydrophoben Carbonsäuren verestert werden, so daß sie in Cremes gelöst oder in biologischen Membranen verankert werden können.

Glucoseester finden sich in therapeutisch wirksamen Pflanzen wie Prunus spec., Rheum spec.oder Thymus spec., welche zur Behandlung von bakteriellen und viralen Infektionen wie Erkältungen und Kopfschmerzen aber auch Beschwerden des Herzens und des Verdauungstraktes eingesetzt werden. Sie spielen unter anderem in der traditionellen chinesischen Medizin eine große Rolle. Dies erklärt, daß die Glucoseester von botanischen Instituten isoliert und bezüglich ihrer Wirksamkeit untersucht wurden (O.M. Abdallah, M.S. Kamel und M.H. Mohamed, *Phytochemistry* **1994**, 37, 1689-1692; J. Budzianowski und L. Skrzypczak, *Phytochemistry* **1995**, 38, 997-1001; M. Ushiyama, S. Kumagai und T. Furuya, *Phytochemistry* **1989**, 28, 3335-3339; Y. Kashiwada, G. I. Nonaka und 1. Nishioka, *Chem. Pharm. Bull.* **1984**, 32, 3461-3470). Wichtige Beispiele für die therapeutische Anwendung der nach dem erfindungsgemäßen Verfahren herstellbaren Ester sind der Effekt auf den Arachidonsäurestofiwechsel in Leukocyten durch Caffeoylglucose (Y. Kimura, H. Okada, S. Nishibe und S. Arichi, *Plant. Med.* **1987**, 53, 148-153), die Verhinderung von Metastasenbildung durch Galloylglucose (N. Ata, T. Oku, M. Hattori, H. Fujii, M. Nakajima und I. Saild, *Oncol. Res.* **1996**, 8, 503-511) sowie die Inhibierung der Herpes simplex Replikation nach Infusion von aromatischen Glucoseestem enthaltenden Infusionen des Verbascum thapsiforme (A. Slagowska, L Zgorniak-Nowosielska und J. Grzybek, *Pol. J. Pharmacol. Pharm.* **1987**, 39, 55-61). Das erfindungsgemäße Verfahren ermöglicht, ausreichende Substanzmengen für pharmakologische Studien und eine breite Anwendung bereitzustellen.

### Beispiel 1: Herstellung von Vitamin C Estern unter kontinuierlicher Entfernung von Methanol

0,9 g Vitamin C (Ascorbinsäure) und 1,35 g Palmitinsäuremethylester in 50 ml Aceton/Methanol (3:1) wurden in einem 2-Halskolben mit aufgesetztem Soxhlet-Extraktor (der mit aktiviertem Molekularsieb befüllt war) mit 0,5 mg immobilisierter Candida antartica B Lipase (SP 435, Hersteller Novo Nordisk) versetzt und unter Rühren (Magnetrührer, 200 UpM) und reduziertem Druck unter Rückfluß erhitzt (ca. 60 °C). Der Reaktionsfortgang wurde dünnschichtchromatographisch verfolgt. Nach Reaktionsende wurden 10 Gewichtsteile warmen (ca. 50 °C) Acetons zugegeben und das Gemisch wurde bei 50 °C filtriert. Das Filtrat wurde auf-10 °C abgekühlt und die in der nachfolgenden Tabelle angegebenen Vitamin C Ester erhalten. Verbindungen **B1** und **B3** wurden zusätzlich durch Extraction mit Chloroform/Wasser (1:1) gereinigt. Alle so erhaltenen Verbindungen wurden mittels NMR-Spektroskopie charakterisiert; das Spektrum von **B3** ist beispielhaft angegeben.

| Verbindung | Reaktionstemperatur | Reaktionszeit | Ausbeute |
|---|---|---|---|
| Ascorbyl-Palmitat **(B1)** | 40°C | 46 h | 79% |
| Ascorbyl-Laurat **(B2)** | 40°C | 34 h | 70 % |
| Ascorbyl-Caproat **(B3)** | 40°C | 18 h | 60 % |

### NMR-Spektrum von B3:

¹³C-NMR (CD₃OD): δ (ppm) = 172,61 (COO im Ring des Ascorbyl-Restes), 170,29 (C-1), 152,39 (COH im Ring des Ascorbyl-Restes), 117,97 (COH bei COO im Ring des Ascorbyl-Restes), 74,92 (CH im Ring des Ascorbyl-Restes), 65,42 (CHOH Ascorbyl-Rest), 33,26 (C-2), 30,99 (C-6), 28,28 (C-4), 28,24 (C-5), 24,26 (C-3), 20,58 (C-7), 13,75 (C-8).

## Patentansprüche

1. Verfahren zur Herstellung von selektiv an der primären OH-Gruppe mit Carbonsäuren veresterten, Zuckerderivaten, **dadurch gekennzeichnet, daß** man das Zuckerderivat ausgewählt aus Aldonsäure und Ascorbinsäure mit einem Carbonsäurealkylester in Gegenwart eines organischen Lösungsmittels unter Katalyse einer Hydrolase, insbesondere einer Lipase oder Esterase, umsetzt.

2. Verfahren nach Anspruch 1, **dadurch gekennzeichnet, daß** die Hydrolase aus den aus Candida antarctica, Humicola lanuginosa, Rhizopus spec., Chromobacterium viscosum, Aspergillus niger, Candida rugosa, Penicillium camembertii, Rhizomucor miehei, Burkholderia spec. oder Pseudomonas spec. erhältlichen Enzymen ausgewählt wird.

3. Verfahren nach Anspruch 1 oder 2, **dadurch gekennzeichnet, daß** die Hydrolase in fester Form, insbesondere auf einem Trägermaterial immobilisiert, eingesetzt wird.

4. Verfahren nach einem der Ansprüche 1 bis 3, **dadurch gekennzeichnet, daß** die Carbonsäuren der allgemeinen Formel R-COOH gehorchen, wobei R ein gegebenenfalls hydroxysubstituierter Alkyl- oder Alkenylrest mit 6 bis 32 C-Atomen oder AR-(CH₂)ₙ ist und AR ein gegebenenfalls alkyl- oder hydroxysubstituierter Phenyl- oder Naphthylrest und n eine Zahl von 0 bis 4 ist.

5. Verfahren nach einem der Ansprüche 1 bis 4, **dadurch gekennzeichnet, daß** die Carbonsäure in Form niederer Alkylester wie der Methyl-, Ethyl-, n-Propyl-, iso-Propyl-, n-Butyl-, sec-Butyl-, iso-Butyl- oder tert-Butylester eingesetzt wird.

6. Verfahren nach einem der Ansprüche 1 bis 5, **dadurch gekennzeichnet, daß** das Molverhältnis zwischen dem Carbonsäureester und dem Zuckerderivat nur möglichst gering von 1 abweicht und insbesondere im Bereich von 0,8 bis 1,2 liegt.

7. Verfahren nach einem der Ansprüche 1 bis 6, **dadurch gekennzeichnet, daß** man organisches Lösungsmittel in 0,1 bis 25 facher, insbesondere 0,5 bis 18 facher Gewichtsmenge an zu veresterndem Zuckerderivat einsetzt.

8. Verfahren nach einem der Ansprüche 1 bis 7, **dadurch gekennzeichnet, daß** man das organische Lösungsmittel aus Dioxan, Acetonitril, Aceton, γ-Butyrolacton, Tetrahydrofuran, tert.-Butanol, tert.-Amylallcohol und 3-Methyl-3-pentanol sowie deren Gemischen auswählt.

9. Verfahren nach einem der Ansprüche 1 bis 8, **dadurch gekennzeichnet, daß** man es bei Temperaturen im Bereich von Raumtemperatur bis 80 °C, insbesondere 60 °C, durchführt.

10. Verfahren nach einem der Ansprüche 1 bis 9, **dadurch gekennzeichnet, daß** man als nichtaktiviertes Carbonsäurederivat einen Ester einsetzt und den aus diesem nach Umsetzung mit dem Zuckerderivat freigesetzten Alkohol mittels azeotroper Destillation aus dem Reaktionsgemisch entfernt.

## Claims

1. A process for the production of sugar derivatives selectively esterified with carboxylic acids at the primary OH group, **characterized in that** the sugar derivative selected from aldonic acids and ascorbic acid is reacted with a carboxylic acid ester in the presence of an organic solvent and a hydrolase, preferably a lipase or esterase, as catalyst.

2. A process as claimed in claim 1, **characterized in that** the hydrolase is selected from the enzymes obtainable from Candida antarctica, Humicola lanuginosa, Rhizopus spec., Chromobacterium viscosum, Aspergillus niger, Candida rugosa, Penicillium camembertii, Rhizomucor miehei, Burkholderia spec. or Pseudomonas spec.

3. A process as claimed in claim 1 or 2, **characterized in that** the hydrolase is used in solid form, more particularly immobilized on a support material.

4. A process as claimed in any of claims 1 to 3, **characterized in that** the carboxylic acids correspond to the general formula R-COOH, where R is an optionally hydroxysubstituted alkyl or alkenyl group containing 6 to 32 carbon atoms or AR-(CH₂)ₙ and AR is an optionally alkyl- or hydroxysubstituted phenyl or naphthyl group and n is a number of 0 to 4.

5. A process as claimed in any of claims 1 to 4, **characterized in that** the carboxylic acid is used in the form of lower alkyl esters, such as the methyl, ethyl, n-propyl, isopropyl, n-butyl, sec-butyl, isobutyl or tert.butyl ester.

6. A process as claimed in any of claims 1 to 5, **characterized in that** the molar ratio between the carboxylic acid ester and the sugar derivative deviates very little from 1 and, more particularly, is in the range from 0.8 to 1.2.1

7. A process as claimed in any of claims 1 to 6, **characterized in that** organic solvent is used in 0.1 to 25 times and more particularly 0.5 to 18 times the quantity by weight of sugar derivative to be esterified.

8. A process as claimed in any of claims 1 to 7, **characterized in that** the organic solvent is selected from dioxane, acetonitrile, acetone, γ-butyrolactone, tetrahydrofuran, tert.butanol, tert.amyl alcohol and 3-methyl-3-pentanol and mixtures thereof.

9. A process as claimed in any of claims 1 to 8, **characterized in that** it is carried out at temperatures in the range from room temperature to 80°C and more particularly at 60°C.

10. A process as claimed in any of claims 1 to 9, **characterized in that** an ester is used as the nonactivated carboxylic acid derivative and the alcohol released therefrom after reaction with the sugar derivative is removed from the reaction mixture by azeotropic distillation.

## Revendications

1. Procédé de préparation de dérivés de sucre sélectivement estérifiés sur le groupe OH primaire avec des acides carboxyliques,
**caractérisé en ce qu'**
on fait réagir le dérivé de sucre choisi parmi l'acide aldonique et l'acide ascorbique avec un ester alkylique d'acide carboxylique en présence d'un solvant organique sous catalyse d'une hydrolase, en particulier d'une lipase ou d'une estérase.

2. Procédé selon la revendication 1,
**caractérisé en ce que**
l'hydrolase est choisie parmi les enzymes que l'on peut obtenir à partir de Candida antartica, Humicola lanuginosa, Rhizopus spec., chromobacterium viscosum, Aspergillus niger, Candida rugosa, Penicillium camembertii, Rhizomucor miehi, Burkholderia spec, ou Pseudomonas spec.

3. Procédé selon la revendication 1 ou 2,
**caractérisé en ce que**
l'hydrolyse est utilisée sous forme solide, en particulier immobilisée sur un support.

4. Procédé selon l'une des revendications 1 à 3,
**caractérisé en ce que**
les acides carboxyliques répondent à la formule générale R-COOH, dans laquelle R représente un radical alkyle ou alcényle éventuellement substitué par un hydroxy, et comportant de 6 à 32 atomes de carbone, ou AR-(CH₂)ₙ et AR est un radical phényle ou naphtyle éventuellement substitué par un alkyle ou un hydroxy, et n est un nombre allant de 0 à 4.

5. Procédé selon l'une des revendications 1 à 4,
**caractérisé en ce que**
l'acide carboxylique est utilisé sous forme d'esters d'alkyle inférieur, comme l'ester méthylique, éthylique, n-propylique, iso-propylique, n-butylique, sec-butylique, iso-butylique ou tert-butylique.

6. Procédé selon l'une des revendications 1 à 5,
**caractérisé en ce que**
le rapport molaire entre l'ester d'acide carboxylique et le dérivé de sucre ne s'écarte que d'aussi peu que possible de 1 et se situe en particulier dans un intervalle allant de 0,8 à 1,2.

7. Procédé selon l'une des revendications 1 à 6,
**caractérisé en ce qu'**
on utilise un solvant organique en une quantité pondérale de 0,1 à 25 fois, en particulier de 0,5 à 18 fois, par rapport au dérivé de sucre à estérifier.

8. Procédé selon l'une des revendications 1 à 7,
**caractérisé en ce qu'**
on choisit le solvant organique parmi le dioxane, l'acétonitrile, l'acétone, la γ-butyrolactone, le tétrahydrofuranne, le tert.-butanol, l'alcool tert.-amylique et le 3-méthyl-3-pentanol ainsi que leurs mélanges.

9. Procédé selon l'une des revendications 1 à 8,
**caractérisé en ce qu'**
on travaille à des températures allant de la température ambiante à 80°C, en particulier à 60°C.

10. Procédé selon l'une des revendications 1 à 9,
**caractérisé en ce qu'**
on utilise un ester comme dérivé d'acide carboxylique non activé, et
on retire du mélange réactionnel l'alcool libéré à partir de celui-ci après réaction avec le dérivé de sucre, au moyen d'une distillation azéotropique.
